# EUROPÄISCHE PATENTANMELDUNG

(11) **EP 2 226 393 A1**
(43) Veröffentlichungstag der Anmeldung: **08.09.2010**
(21) Anmeldenummer: 09154527.7
(22) Anmeldetag: 06.03.2009
(51) Int. Cl.: C12Q 1/68

(54) **CHK2 Polymorphismus als Krebsmarker**

(71) Anmelder: Universität Duisburg-Essen, 45145 Essen (DE)
(72) Erfinder: Siffert, Winfried, 45884 Gelsenkirchen (DE); Riemann, Kathrin, 45145 Essen (DE)
(74) Vertreter: Cohausz & Florack

(57) **Zusammenfassung**

Die Erfindung betrifft die Verwendung von Genveränderungen im humanen Gen *CHK2* (*CHEK2*), das für die Checkpointkinase 2 kodiert, zur Vorhersage des Risikos und des Verlaufs von Krebserkrankungen und zur Vorhersage des Ansprechens auf pharmakologische oder nicht-pharmakologische Therapiemaßnahmen zur Behandlung von Krebserkrankungen sowie zur Vorhersage unerwünschter Arzneimittelwirkungen. Ferner betrifft die Erfindung die Bereitstellung von einzelnen Genvarianten, mit deren Hilfe weitere für die oben genannten Zwecke verwendbaren Genveränderungen detektiert und validiert werden können. Solche Genveränderungen können darin bestehen, dass im Promotor von *CHK2* an Position -7161 eine Substitution von Guanin zu Adenin vorliegt, an Position -7235 eine Substitution von Cytosin zu Guanin, an Position -10532 eine Substitution von Guanin zu Adenin vorliegt oder an Position -10621 bis -10649 eine Deletion von 29 Basenpaaren vorliegt.

## Beschreibung

Die Erfindung betrifft ein diagnostisches Verfahren zur Ermittlung einer geeigneten Therapie zur Behandlung einer Krebserkrankung.

Ein wesentlicher Aspekt bei der Behandlung von Krebs ist zunächst das Erstellen einer genauen Diagnose. Dies führt zu Kenntnissen darüber, in welchem Stadium sich die Krebserkrankung befindet, was auch über die nachfolgenden Therapiemodalitäten entscheidet, z.B. chirurgische Entfernung des Tumors mit ggfls. Bestrahlung und pharmakologischer Therapie, eventuell auch unter Einsatz physikalischer Verfahren (z.B. Hyperthermie). Das klassische Verfahren zur Diagnosestellung beinhaltet das sogenannte "Staging" und "Grading". Das Staging beschreibt die Tumorgröße und die Invasivität und untersucht, ob Lymphknoten befallen sind und ob Fernmetastasen vorhanden sind (z.B. TMN-System). Beim Grading werden die Tumorzellen histologisch untersucht, wobei hoch differenzierten Tumorzellen ein niedrigeres Malignitätspotential zugeordnet wird als schwach differenzierten oder entdifferenzierten Zellen. Bei dieser Einteilung ist die individuelle Vorhersagekraft für den einzelnen Patienten limitiert, und es entspricht der klinischen Erfahrung, dass Patienten mit gleichem Tumorstadium deutlich unterschiedliche Krankheitsverläufe zeigen und unterschiedlich auf eine Therapie ansprechen, was sich letztendlich in drastisch unterschiedlichen Überlebenszeiten zeigt.

Aus diesem Grunde ist es für die klinische Praxis erforderlich, zusätzliche und präzisere Marker bereitzustellen, die eine verbesserte Individualprognose einer Krebserkrankung erlauben. Dies kann geschehen über die Einbeziehung histochemischer Marker, wie dies beim Mammakarzinom im Rahmen der Untersuchung der Expression von Östrogenrezeptoren erfolgt.

Ein alternativer Weg ist die Suche nach Gendefekten, z.B. somatischen Mutationen in Tumorsuppressorgenen. Neuere Ansätze versuchen den Krankheitsverlauf mit Hilfe von Genexpressionsprofilen vorherzusagen. Ein weiteres Problem bei der Krebsbehandlung ist die Vorhersage der Wirksamkeit von Krebsmedikamenten, der individuell optimalen Dosierung oder Therapiedauer, aber auch die Vorhersage des Auftretens schwerwiegender und bedrohlicher Nebenwirkungen. Beispielsweise können genetische Polymorphismen im Gen *UGT1A1* dafür verantwortlich gemacht werden, dass bei einigen Patienten unter Therapie mit Irinotecan schwerwiegende Nebenwirkungen auftreten. Eine vorherige Genanalyse kann solche Patienten vor Therapiebeginn identifizieren und eine Dosisanpassung ermöglichen. Daneben sind Therapien mit in biotechnologischen Verfahren hergestellten Medikamenten teuer und werden zukünftig auf die Patienten beschränkt werden, bei denen eine Wirksamkeit auf Grund der individuellen Disposition wahrscheinlich erscheint.

### Grundlegende Eigenschaften von malignen Tumoren

Krebszellen sind gekennzeichnet durch einen Verlust der Kontaktinhibition und unkontrolliertes Zellwachstum. Ausgelöst werden solche Veränderungen spontan oder durch Noxen, sog. Kanzerogene, welche das Erbgut schädigen. Zu solchen Noxen gehören viele Chemikalien, Tabakrauch, aber auch UV-Licht. Daneben spielen genetische Faktoren bei der Krebsentstehung eine herausragende Rolle. Kennzeichnend für Krebszellen ist neben ihrem ungehemmten Wachstum auch die Neigung, Tochtergeschwülste (Metastasen) in anderen Organen abzusiedeln. Die Ausbreitung der Metastasen erfolgt regelmäßig über die Blutbahn oder über Lymphgefäße. Krebserkrankungen sind bei einem Großteil der Fälle nicht heilbar und tödlich. Therapeutisch wird versucht, den Ausgangstumor und Metastasen operativ zu entfernen. Daneben können Tumore bestrahlt werden. Mittels so genannter Zytostatika, Antikörper gegen bestimmte Proteine oder Oberflächenmarker oder immunmodulierender Substanzen (Zytokine, Interferone) wird versucht, die sich schnell teilenden Krebszellen abzutöten oder in den programmierten Zelltod (Apoptose) zu überführen.
Es ist von medizinisch großer Relevanz, Prognosefaktoren für den Verlauf von Krebserkrankungen zu definieren, die Auskunft über das Ansprechen auf bestimmte Therapieformen geben oder die generell prädiktiv für das Auftreten von Metastasen, die Tumorprogression und das Überleben sind.

Ganz offensichtlich gibt es eine Vielzahl individueller, unerkannter biologischer Variablen, die den Verlauf einer Tumorerkrankung unabhängig von Staging und Grading determinieren. Zu solchen Faktoren gehören genetische Wirtsfaktoren. Daneben ist es wünschenswert, genetische Marker zu entwickeln, die prädiktiv für das Auftreten von Tumoren sind. Solche Marker erfüllen die Funktion, die betroffenen Individuen frühzeitig weiteren Screeningmaßnahmen (Serologie, Röntgen, Ultraschall, MRT etc.) zuzuführen. Damit können Krebserkrankungen im Frühstadium erkannt und therapiert werden, wobei die Heilungs- und Überlebenschancen bei Tumoren im Frühstadium wesentlich besser sind als bei fortgeschrittenen Tumoren.

### Bedeutung von DNA- Reparaturmechanismen

Durch DNA-Reparatur-Mechanismen können Zellen schadhafte Veränderungen der DNA-Struktur beseitigen. Solche Schäden in der DNA können spontan im Verlauf der DNA-Replikation oder durch die Einwirkung mutagener Substanzen, extremer Wärme oder ionisierender Strahlung verursacht werden. DNA-Schäden können dazu führen, dass die Replikation der DNA für die Mitose falsch erfolgt, Proteine nicht mehr bzw. falsch synthetisiert oder wichtige Chromosomenbereiche nach Doppelstrangbrüchen abgespalten werden. Bringen die komplexen Reparaturmechanismen der Zelle keinen Erfolg, so sammeln sich in wachsenden und ruhenden somatischen Zellen so viele Fehler an, dass die normalen Zellfunktionen gestört sind. In einer Keimzelle wären die Tochterzellen nicht mehr lebensfähig, was zu einer Inaktivierung der Zelllinie führt: die Zelle bzw. die zweite bis dritte nachfolgende Generation verliert ihre Teilungsfähigkeit und stirbt. Im Zuge der Zellzykluskontrolle können Kontrollproteine eine Zelle bzw. deren DNA als defekt erkennen und einen Zyklusarrest oder den programmierten Zelltod (Apoptose) einleiten.

### Bedeutung der Checkpointkinase 2

Die wichtigste Aufgabe einer Zelle ist, die genomische Integrität aufrecht zu erhalten. Aus diesem Grund existieren diverse Kontrollmechanismen, die sicherstellen, dass sämtliche Prozesse innerhalb des Zellzyklus korrekt vollendet werden. Diese Kontrollmechanismen werden als "Checkpoints^{[1]}" bezeichnet. Dabei handelt es sich nicht um einen fest definierten Punkt, wie das Wort selbst impliziert, sondern um eine Reaktionskaskade, die unter bestimmten Umständen initiiert werden kann.

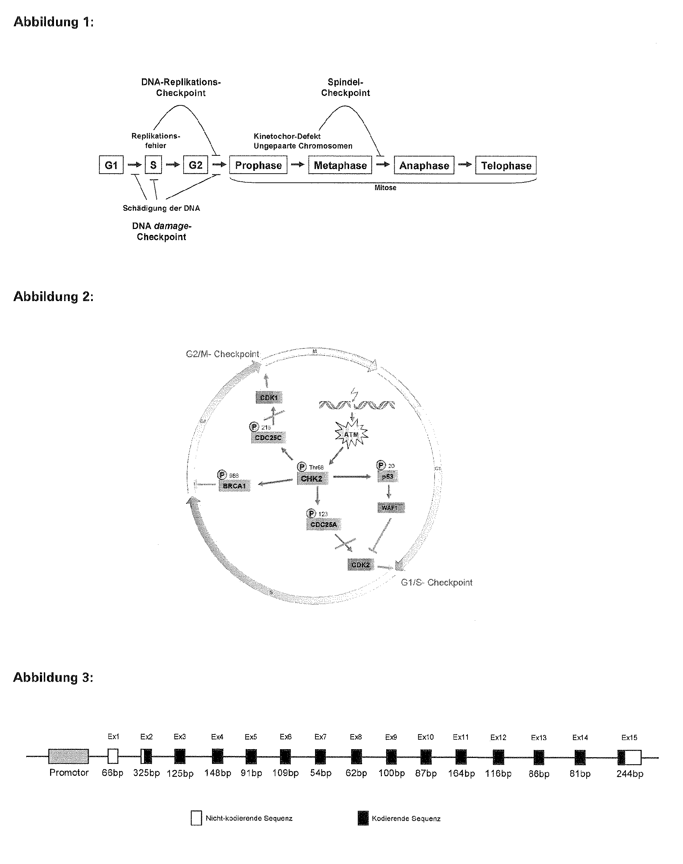

Bisher wurden mehrere Zellzyklus-Checkpoints charakterisiert. Die am besten untersuchten Checkpoints in Säugetieren sind in Abbildung 1 dargestellt. Zum einen gibt es den DNA *damage-*Checkpoint, der durch Schädigung der DNA in unterschiedlichen Zellzyklus-Phasen aktiviert werden kann. Diese Schädigung kann sowohl durch exogene Ursachen, wie Strahlung, als auch durch endogene Vorgänge, z.B. spontane Mutationen, hervorgerufen werden. Zum anderen tritt durch eine nicht vollständige oder fehlerhafte Replikation der DNA der Replikations-Checkpoint in Kraft. Der Spindel-Checkpoint überwacht die Bildung der bipolaren Spindel, die Anlagerung der Kinetochore und die Neubildung der Zentromerstrukturen.

Solange diese Vorgänge nicht vollständig abgeschlossen sind bzw. die Schädigung nicht behoben ist, wird der Eintritt der Zelle in die nächste Zellzyklusphase inhibiert, um sicher zu stellen, dass die genomische Integrität der Zelle erhalten bleibt.

Die Checkpointkinase 2 ist in essentielle Kontrollmechanismen im Zellzyklus involviert, die sicherstellen, dass die Weitergabe von Fehlern an die Tochterzellen minimiert wird. Die maßgeblichen CHK2-Reaktionskaskaden, die bei verschiedenen Checkpoints aktiviert werden, sind in Abbildung 2 dargestellt. Aufgrund von DNA-Schädigungen wird ATM (*ataxia-telangiectasia, mutated*) phosphoryliert und damit aktiviert. ATM wiederum aktiviert CHK2 über eine Phosphorylierung an einem Threonin (Thr) an Aminosäure-Position 68. Diese Phosphorylierung ist eine Voraussetzung für die Fähigkeit zur Dimerbildung, über die sich CHK2 autophosphorylieren kann. Nur auf diese Weise kann CHK2 voll aktiviert vorliegen und ihre Effektoren aktivieren, über die in defekten Zellen ein Arrest im Zellzyklus während der G1-, S- oder G2/M-Phase, Aktivierung des DNA-Reparatursystems oder Apoptose induziert werden können.

Zu den Effektoren von CHK2 gehört p53 (*tumor protein 53*), das durch CHK2 an Serin (Ser) 20 phosphoryliert und dadurch stabilisiert wird. P53 reguliert daraufhin Faktoren, die in die Reparatur von DNA-Schäden, Apoptose und die Kontrolle des Zellzyklus involviert sind positiv. Auch BRCA1 (*breast cancer 1 gene*) gehört zu den CHK2-Substraten. Es wird durch CHK2 an Ser988 phosphoryliert und dadurch aus seinem Komplex mit CHK2 entlassen, was wiederum zum Stop des Zellzyklus führt, um geschädigte DNA zu reparieren. CDC25C (*cell division cycle 25C*) wird ebenfalls durch CHK2 an Ser216 phosphoryliert, wodurch seine eigene Phosphatase-Aktivität gehemmt wird und es zytoplasmatisch abgebaut wird. Cdk1 (*cyclin-dependent kinase 1*) kann dann nicht aktiviert werden und es wird verhindert, dass Zellen mit geschädigter DNA in die Mitose eintreten. Zudem wird durch CHK2 CDC25A an Ser123 phosphoryliert, woraufhin es ubiquitiniert und Proteasom-abhängig degradiert wird. Dieses Protein spielt eine wichtige Rolle für das Fortschreiten der Zelle im Zellzyklus, was allerdings durch den Abbau gehemmt wird).

CHK2 spielt eine wichtige Rolle bei der durch DNA-Schädigung initiierten Signaltransduktion. Die physiologische Bedeutung von CHK2 wurde untersucht, indem *knock* out-Mäuse generiert wurden. Sowohl *Chk2*^{*+*/*+*}- als auch *Chk2*^{-/-}-Mäuse waren lebensfähig. Allerdings zeigten sich nach einer Bestrahlung mit sublethalen Dosen (8 Gy) unterschiedlich lange Überlebenszeiten: Das mediane Überleben von Wildtyp- und *Chk2*^{*+*/*-*}-Mäusen war bedeutend kürzer als das von *Chk2*^{-/-}-Mäusen. Somit zeigte sich bei den *Chk2*^{-/-}-Mäusen eine Radioresistenz, die durch eine verringerte Strahlungsinduzierte Apoptoserate hervorgerufen wird. Während der G2/M-Checkpoint bei *Chk2*^{-/-}-Mäusen nicht beeinträchtigt war, kann der G1/S-Checkpoint zwar initiiert, aber nicht aufrecht gehalten werden. Dies geht auf eine verringerte transkriptionelle Aktivität von p53 zurück. Zudem wurden Tumore nur bei *Chk2*^{-/-}-Mäusen entwickelt im Gegensatz zu Mäusen, die noch mindestens ein funktionsfähiges *Chk2*-Allel besitzen

Der vorliegenden Erfindung liegt daher die Aufgabe zu Grunde, eine Methode bereitzustellen, die eine bessere Prognose des natürlichen Verlaufs einer Krebserkrankung und das Ansprechen auf jedwede Therapieform erlaubt. Insbesondere soll diese Methode auch dazu in der Lage sein, diejenigen Patienten zu erkennen, bei denen gesteigerte DNA-Reparaturmechanismen eine Krebstherapie erschweren.

Gelöst wird diese Aufgabe durch ein In vitro-Verfahren zur Vorhersage des Krankheitsrisikos für eine Krebserkrankung, des Krankheitsverlaufs, des Arzneimittelnutzen und Arzneimittelrisikos bei der Behandlung einer Krebserkrankung, in dem man in einer Patientenprobe nach einer oder mehreren Genveränderung/en in der Promotorregion des Gens CHK2 auf dem Humanchromosom 22q12.1 sucht und die Genveränderung ausgewählt ist aus dem Polymorphismus -7161G>A, dem Polymorphismus -7235C>G, dem Polymorphismus -10532G>A und dem Polymorphismus -10649-(-10621)del29. Gegenstand der Erfindung ist auch die Verwendung vorgenannter Polymorphismen für die zuvor angegebenen Zwecke.

Die Erfindung zielt folglich darauf ab
a. funktionsverändernde genomische Polymorphismen im Promotor des Gens *CHK2* bereitzustellen, die entweder zur Änderung der Proteinexpression oder zur Änderung der Expression von Spleißvarianten führen, oder
b. die zum Auffinden und/oder Validieren weiterer Polymorphismen bzw. Haplotypen im Gen *CNK2* geeignet sind,
c. Polymorphismen bereitzustellen, die geeignet sind, generell Krankheitsrisiken und -verläufe vorherzusagen,
d. Polymorphismen bereitzustellen, die geeignet sind, generell Ansprechen auf Pharmaka und Krebstherapien, insbesondere CHK2-Inhibitoren, und Nebenwirkungen vorherzusagen,
e. Polymorphismen bereitzustellen, die geeignet sind, generell die Wirkung anderer Therapieformen vorherzusagen (wie Bestrahlung, Wärme, Kälte).

Wegen der grundlegenden Bedeutung von CHK2 für die Aufrechterhaltung der genomischen Integrität sind solche Polymorphismen geeignet, generell Erkrankungsrisiken bzw. Krankheitsverläufe bei Krebserkrankungen vorherzusagen bzw. Therapieansprechen/Therapieversagen oder unerwünschte Nebenwirkungen für alle Pharmaka oder nicht-pharmakologische Therapien vorherzusagen.

### Nachweis von Polymorphismen im Promotor des Gens CHK2

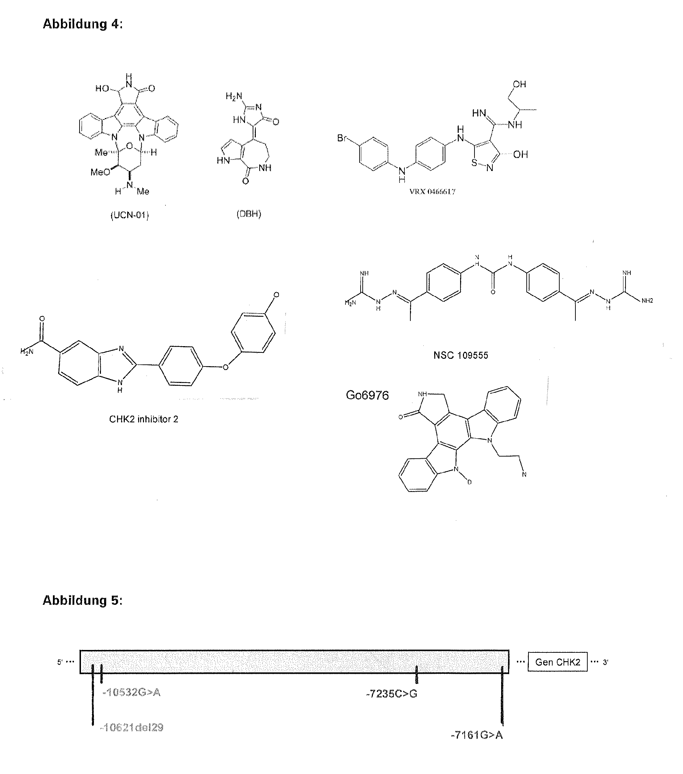

In der Promotorregion von *CHK2* sind drei Polymorphismen bekannt und in allgemein zugänglichen Datenbanken aufzufinden. Durch die systematische Sequenzierung von DNA-Proben von Menschen wurden diese drei Polymorphismen nachgewiesen und validiert: -7161G>A (rs2236141), -7235C>G (rs2236142) und -10532G>A (rs5762767) (Abbildung 5). Hierzu wurden Gensequenzen des Promotorbereichs von *CHK2* mittels PCR-Reaktion amplifiziert und gemäß der Methode nach Sanger sequenziert. Die dazu erforderlichen Verfahren, z.B. das Ableiten von für die PCR-Reaktion erforderlichen Primerpaaren und die Auswahl von Sequenzier-Primern sind dem Fachmann geläufig. Hierbei wurde ein neuer Polymorphismus gefunden, bei dem es sich um eine Deletion von 29 Basenpaaren handelt (-10649-(-10621)del29, keine dbSNP-Kennung vorhanden) (Abbildung 5). Die Nummerierung dieser SNPs erfolgt in der Weise, dass dem Nukleotid A des Startcodons ATG die Nummer +1 zugeordnet wird. Da es der Konvention entsprechend die Nummer 0 nicht gibt, ist dem vor dem A des Startcodons ATG liegenden Nukleotid die Nummer-1 zugeordnet.

Der Nachweis dieser Polymorphismen im Sinne ihrer erfindungsgemäßen Verwendung kann mit beliebigen, dem Fachmann geläufigen Verfahren unternommen werden, z.B. direkte Sequenzierung, PCR mit nachfolgender Restriktionsanalyse, reverse Hybridisierung, Dot-blot- oder Slot-blot-Verfahren, Massenspektrometrie, Taqman□- oder Light-Cycler□-Technologie, Pyrosequencing□, Invader□-Technologie, Luminex-Verfahren. Ferner können diese Genpolymorphismen gleichzeitig nach Mulitplex-PCR und Hybridisierung an einen DNA-Chip detektiert werden.

Generell können alle Zellen des menschlichen Körpers maligne entarten und zu einer Krebserkrankung führen. Die hier und im Weiteren gemachten Ausführungen beschreiben generelle Mechanismen der Tumorprogression, der Metastasierung und des therapeutischen Ansprechens. Insofern gelten die hier beschriebenen Mechanismen und Ansprüche für alle Tumore des Menschen. Die Folgenden sind nur beispielhaft genannt.

Tumoren des Urogenitaltrakts wie das Nierenzellkarzinom, das Prostatakarzinom und das Seminom; Tumoren der weiblichen Geschlechtsorgane wie das Mammakarzinom, das Korpuskarzinom, das Ovarialkarzinom, das Zervixkarzinom; Tumoren des Gastrointestinaltraktes wie das Mundhöhlenkarzinom, das Ösophaguskarzinom, das Magenkarzinom, das Leberkarzinom, das Gallengangskarzinom, das Pankreaskarzinom, das Kolonkarzinom, das Rektumkarzinom; Tumore des Respirationstrakts wie das Kehlkopfkarzinom, das Bronchialkarzinom; Tumore der Haut wie das maligne Melanom, das Basaliom und das T-Zell-Lymphom; Tumorerkrankungen des blutbildenden Systems wie Hodgkin- und Non-Hodgkin-Lymphome, akute und chronische Leukämien, Plasmozytom; Tumorerkrankungen des Gehirns bzw. des Nervengewebes wie Glioblastom, Neuroblastom, Medulloblastom, meningeales Sarkom, Astrozytom sowie Weichteiltumore wie Sarkome und Kopf-Hals-Tumore.

### Das Gen CHK2

Das humane Gen *CHK2* ist auf Chromosom 22q12.1 lokalisiert (Gene bank accession number NM_007194) und kodiert für ein 65 kD großes Protein, das nukleär exprimiert wird. An dieser Stelle soll darauf hingewiesen werden, dass das Gen sowohl die Bezeichnung *"CHK2"* als auch die Bezeichnung *"CHEK2"* trägt. Im Folgenden wird hier die Bezeichnung *"CHK2"* verwendet. Eine schematische Darstellung der Genstruktur findet sich in Abbildung 3. Die aktive Promotorregion von *CNK2* wurde bereits charakterisiert. Die Promotorsequenz enthält zahlreiche Bindestellen für den Transkriptionsfaktor SP1, dessen Bindung die transkriptionelle Aktivität verstärkt. Eine positive Regulation von *CHK2* wurde ebenfalls durch eine Bindung von NF-Y an CCAAT-Boxen beobachtet

### Somatische Mutationen in CHK2

*CHK2* wird als potentielles Tumorsuppressor-Gen betrachtet, da es eine große Rolle beim Checkpoint Arrest nach DNA-Schädigung spielt und verschiedene Tumorsuppressorgene Substrate von CHK2 sind. Somatische Mutationen in diesem Gen konnten bisher in einigen Patienten mit sporadischen Tumoren nachgewiesen werden, z.B. kolorektale Tumore, Lungen-, Prostata- und Mammakarzinom, sowie bei Patienten mit dem Li-Fraumeni-Syndrom, einem Multitumor-Phänotyp. Im Unterschied zu *single nucleotide polymorphisms* (SNPs) werden diese Mutationen bei den entsprechenden Patienten beispielsweise nicht in peripheren Blutzellen gefunden.

### Risiko für Tumorerkrankungen durch Veränderungen in CHK2

Es ist bisher mehrfach gelungen, genetische Veränderungen in *CHK2* bei verschiedenen Tumorerkrankungen nachzuweisen und diese mit dem Erkrankungsrisiko zu assoziieren. Dabei handelte es sich nicht um häufige genetische Varianten, sondern um seltene Mutationen (Häufigkeit <1%), die in großen Patientenkollektiven öfter nachweisbar waren als in gesunden Kontrollen.
Bisher ist nur eine Studie veröffentlicht, in der nicht nur diese seltenen Mutationen, sondern auch häufig auftretende SNPs untersucht wurden. Dabei wurde auch der Promotorpolymorphismus -7161G>A (rs2236141) auf das Risiko für das Entstehen eines Mammakarzinoms analysiert. Allerdings konnte keine Assoziation nachgewiesen werden (Baynes et al. Common variants in the ATM, BRCA1, BRCA2, CHEK2 and TP53 cancer susceptibility genes are unlikely to increase breast cancer risk. 2007 Breast Cancer Res 9:R27).

### CHK2-Inhibitoren als Chemotherapeutika

Da Checkpoints in viele regulatorische Kaskaden involviert sind, sind sie ein geeignetes Ziel für Krebstherapeutika. Dazu tragen bestimmte Eigenschaften der Checkpoint-Proteine bei: (1) das komplexe Signaltransduktionssystem von Checkpoints bietet eine Vielzahl von Angriffszielen, (2) in gesunden Zellen scheinen einige Checkpoints relativ bedeutungslos zu sein, was die Toxizität der Inhibitoren stark reduziert, (3) die Restoration defekter Checkpoints könnte zu einer Verlangsamung des Zellwachstums führen, (4) als Signaltransduktionssystem unterliegen Checkpoints der Adaption, die unterbrochen werden könnte und (5) die Wiederherstellung beeinträchtigter Checkpoints könnte die Apoptoserate von Krebszellen erhöhen und somit ihre Sensitivität gegenüber bestimmten Stoffen erhöhen.

Im Gegensatz zu diesen Punkten, die am ehesten über einen Gentherapieansatz zu verwirklichen sind, stellen zwei weitere Eigenschaften von Checkpoints leichter realisierbare Angriffspunkte dar. Zellen mit defekten Checkpoints zeigen entweder eine stark erhöhte Sensitivität oder eine erhöhte Resistenz gegenüber Strahlung und zytotoxischen Substanzen. Die Inhibition von CHK2 scheint besonders p53- defiziente Zellen für DNA-schädigende Agenzien zu sensitivieren und dabei das Normalgewebe vor einer Schädigung und damit Nebenwirkungen zu schützen. Daher gehört die Hemmung von CHK2 zu einem aussichtsreichen Ansatz für die Entwicklung neuer Krebstherapeutika.

Diverse CHK2-Inhibitoren sind bereits bekannt bzw. entwickelt worden. Neben den eher unspezifischen Hemmstoffen UCN-01 und DBH (Debromohymenialdisin), stehen nun auch spezifische potente CHK2-Inhibitoren zur Verfügung. Dazu gehören CHK2 inhibitor 2 (2-(4-(4-chlorophenoxy)phenyl)-1H-benzo(d)imidazol-5-carboxamid), VRX0466617 (5-(4-(4-bromophenylamino)phenyl-amino)-3-hydroxy-N-(1-hydroxypropan-2-yl)isothiazol-4-carboximidamide) und NSC 109555 ((2E,2'E)-2,2-(1,1'-(4,4'-Carbonyl-bis(azanediyl)bis(4,1-phenylen))bis(ethan-1-yl-1-yliden))bis(hydrazincarboximidamid), die zur Reduktion von Nebenwirkungen unter Strahlentherapie beitragen könnten (Abbildung 4).

### CHK2-Aktivatoren als Chemotherapeutika

Unter Umständen könnten aber auch CHK2-Aktivatoren eine Alternative bei der Krebstherapie darstellen. CHK2 spielt eine Rolle bei der Unterdrückung der Onkogenese und ihre Aktivierung kann Tumorzellen dazu bringen, den proliferativen Status aufzugeben und abzusterben, wenn keine DNAschädigenden Agenzien vorhanden sind. Zudem wird dadurch ein starker G2-Arrest induziert. Die Aktivierungsstrategie könnte einen Nachteil der Inhibitoren ausgleichen: Tumore sind sehr heterogen und deshalb mag es nicht immer ausreichen, nur eine Signalkaskade auszuschalten. Dieser Heterogenität könnte mit der Überaktivierung konservierter Zellzyklus-Mechanismen, die viele Tumorzellarten teilen, entgegengewirkt werden.

### Verteilung der -7161G>A, -7235C>GT, -10532G>A und -10649-(-10621)del29-Polymorphismen, Nachweis von Haplotypen und Verwendung dieser Genotypen zum Auffinden weiterer relevanter Polymorphismen und Haplotypen.

Hierzu wurden unterschiedliche DNA-Proben von Kaukasiern genotypisiert. Das Ergebnis ist in der folgenden Tabelle dargestellt:

| **Polymorphismus** | **Genotypen** | | |
|---|---|---|---|
| -7161G>A | GG: 188 | GA: 43 | AA: 4 |
| -7235C>G | CC: 25 | CG: 88 | GG: 121 |
| -10532G>A | GG: 188 | GA: 43 | AA: 4 |
| -10649-(-10621)del29 | II: 56 | ID: 109 | DD: 56 |

| | | | |
|---|---|---|---|
| I: Insertion, D: Deletion | | | |

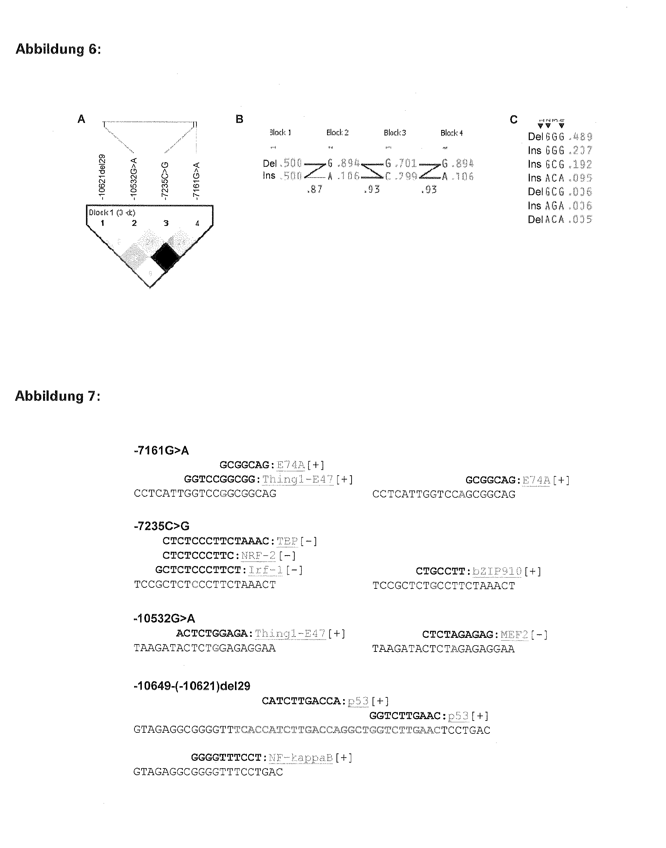

Weitere Analysen zeigten bei diesen DNA-Proben von gesunden Kaukasiern ein Kopplungsungleichgewicht zwischen bestimmten Polymorphismen. Unter Kopplungsungleichgewicht versteht man das Auftreten von Alleikombinationen (Haplotypen), die statistisch eindeutig häufiger oder seltener gemeinsam vorkommen, als dies bezogen auf ihre Frequenz zu erwarten wäre. Dabei stellte sich heraus, dass die Polymorphismen -7161G>A und -10532G>A vollständig aneinander koppeln. Die Polymorphismen -7235C>G und -10649-(-10621)del29 dagegen koppeln nicht aneinander und nur eingeschränkt an die beiden anderen Varianten (Abbildung 6A und B). Die Güte der Kopplung wird durch die Werte D' und r² gekennzeichnet. Dabei spricht man bei D'=1 und r²=1 von einer signifikanten Kopplung. Je näher beide Werte bei 1 liegen, desto enger ist das Kopplungsungleichgewicht. Die Berechnung der Haplotypen, die aus diesen vier Polymorphismen konstruiert werden können, ergab sieben verschiedene Allelkombinationen. Es existiert kein präferentieller Haplotyp, der sich aus diesen Promotorvarianten ergibt (Abbildung 6C). Um alle möglichen Kombinationen zu bestimmen, ist der Nachweis von mindestens drei der vier Polymorphismen notwendig.

Ein Gegenstand der Erfindung ist es, dass diese neuen Polymorphismen dazu benutzt werden können, um weitere relevante genomische Genveränderungen in *CHK2* oder benachbarten Genen zu detektieren und zu validieren, die beispielsweise mit Genotypen im Gen *CHK2* im Kopplungsungleichgewicht stehen. Dies können auch Gene sein, die ebenfalls auf Chromosom 22 liegen, aber in großer Entfernung vom Gen *CHK2.* Hierzu wird folgendermaßen vorgegangen:
1. Für bestimmte Phänotypen (wie zelluläre Eigenschaften, Krankheitszustände, Krankheitsverläufe, Arzneimittelansprechen) wird zunächst eine Assoziation mit den Polymorphismen -7161G>A, - 7235C>G, -10532G>A und -10649-(-10621)del29 hergestellt, wobei diese Assoziationen für jeden Genotyp einzeln oder unter Verwendung aller Permutationen der Haplotypen aufgestellt werden können.
2. Für neu detektierte Genveränderungen in *CHK2* oder benachbarten Genen wird untersucht, ob bereits bestehende Assoziationen unter Verwendung oben beschriebener Geno- oder Haplotypen verstärkt oder abgeschwächt werden.

Die Abbildungen werden nachfolgend kurz erläutert.
- **Abbildung 1:**: Schematische Darstellung des Zellzyklus mit den wichtigsten Checkpoints
- **Abbildung 2:**: Graphische Darstellung der Reaktionskaskade an den von CHK2-regulierten Check-points
- **Abbildung 3:**: Intron/Exon-Struktur des humanen Gens *CHK2* (nicht maßstabsgetreu)
- **Abbildung 4:**: Strukturformeln einiger CHK2-Inhibitoren
- **Abbildung 5:**: Schematische Darstellung der Polymorphismen im Gen *CHK2* (nicht maßstabsgetreu)
- **Abbildung 6:**: Kopplungsanalysen der Promotor-Polymorphismen von *CHK2* mit dem Programm Haploview; A-Graphische Darstellung der Kopplung der Polymorphismen untereinander. Schwarze Quadrate zeigen r² =1 an, graue Quadrate r²<0.5 und hellgraue Quadrate r²<0.1; B- Häufigkeiten und Kopplungsmöglichkeiten der einzelnen Allele; C- Häufigkeiten der konstruierten Haplotypen; Allele, die mit einem Dreieck gekennzeichnet sind, werden als sog. *haplotype-tagging alleles* bezeichnet, d. h. diese Allele müssen bestimmt werden, um die jeweiligen Haplotypen zu bestimmen.
- **Abbildung 7:**: Putative Bindungsstellen für Transkriptionsfaktoren im Promotor des Gens *CHK2*; Rot markierte Basen stellen die Allele des jeweiligen Polymorphismus dar.
- **Abbildung 8:**: Ergebnis des Electrophoretic Mobility Shift Assays (EMSA) mit Konstrukten, die die verschiedenen Allele des -7235C>G-Polymorphismus von *CHK2* enthalten. Nach Zugabe von Zellkernextrakt erkennt man eine vermehrte Bindung von Kernprotein and das C-Allel. Die Bindung wird durch die Gegenwart eines verdrängenden Oligonukleotids spezifisch gehemmt.
- **Abbildung 9:**: Ergebnis des Electrophoretic Mobility Shift Assays (EMSA) mit Konstrukten, die die verschiedenen Allele des -10649-(-10621)del29-Polymorphismus von *CHK2* enthalten.
Nach Zugabe von Zellkernextrakt erkennt man, dass beide Allele zu einer Bindung eines Transkriptionsfaktors führen, der jeweils durch das andere Allel kompetitiert werden kann. Hierbei handelt es sich aber um unterschiedliche Transkriptionsfaktoren.
- **Abbildung 10:**: *CHK2*-Promotoraktivität in Abhängigkeit vom -10649-(-10621)del29-Polymorphismus bestimmt durch SEAP-Reporter-Assays; **: p<0.01.
- **Abbildung 11:**: Expression von CHK2-mRNA in Abhängigkeit vom -7161G>A-Polymorphismus. Dargestellt ist der Quotient CHK2-/β-Actin-mRNA. A: Mammakarzinom, B: CLL; **: p<0.01; *: p<0.05
- **Abbildung 12:**: Expression von CHK2-mRNA in Abhängigkeit vom -7235C>G-Polymorphismus. Dargestellt ist der Quotient CHK2-/β-Actin-mRNA. A: Mammakarzinom, B: CLL; *: p<0.05
- **Abbildung 13:**: Expression von CHK2-mRNA in Abhängigkeit vom -10649-(-10621)del29-Polymorphismus bei Mammakarzinom-Patientinnen. Dargestellt ist der Quotient CHK2-/β-Actin-mRNA.
- **Abbildung 14:**: Kaplan-Meier-Analyse zum Überleben von Patienten mit kolorektalem Karzinom abhängig vom Genotyp des -7161G>A-Polymorphismus.
- **Abbildung 15:**: Kaplan-Meier-Analyse zum Überleben von Patienten mit chronischer lymphatischer Leukämie abhängig vom Genotyp des -7235C>G-Polymorphismus; *: p<0.05.
- **Abbildung 16:**: Kaplan-Meier-Analyse von Patienten mit Nierenzellkarzinom der Stadien 3 und 4. A: Überleben abhängig vom Genotyp des -7161 G>A-Polymorphismus, B: Progressionsfreies Überleben abhängig vom Genotyp des -7161G>A-Polymorphismus, C: Überleben abhängig vom -7235C>G-Polymorphismus; ***: p<0.001; *: p<0.05.
- **Abbildung 17:**: Kaplan-Meier-Analyse zum Überleben von Patientinnen mit Mammakarzinom abhängig vom Genotyp des -10649-(-10621)del29-Polymorphismus.
- **Abbildung 18:**: Kaplan-Meier-Analyse zum Überleben von Patienten mit Glioblastom. A: Überleben, B: Rezidivfreies Überleben abhängig vom Genotyp des -10649-(-10621)del29-Polymorphismus, C: Überleben, D: Rezidivfreies Überleben abhängig vom -7235C>G-Polymorphismus; **: p<0.01; *: p<0.05
- Abbildung 19:: Kaplan-Meier-Analyse zum Überleben von Patientinnen mit Prostatakarzinom abhängig vom Genotyp des -7161G>A-Polymorphismus; **: p<0.01

### Funktionelle Bedeutung der Promotor-Polymorphismen im Gen CHK2

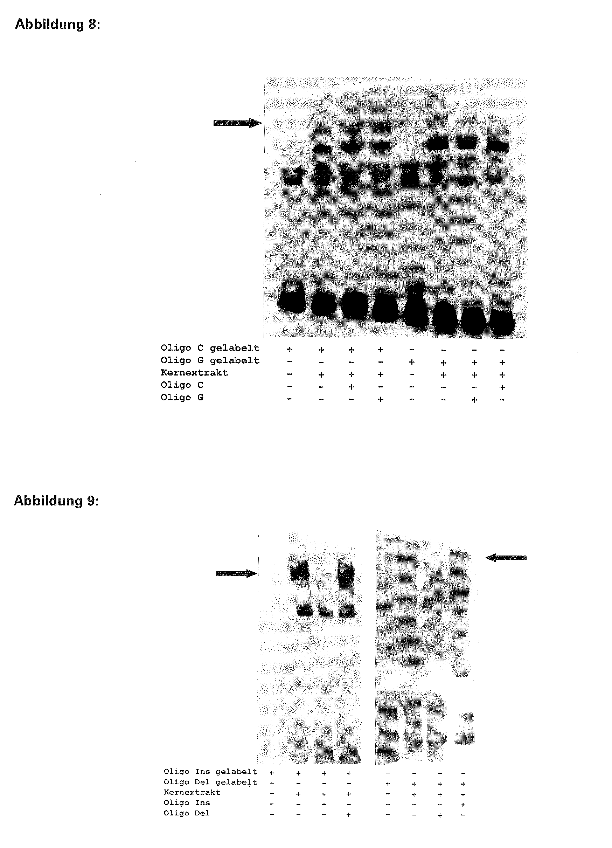

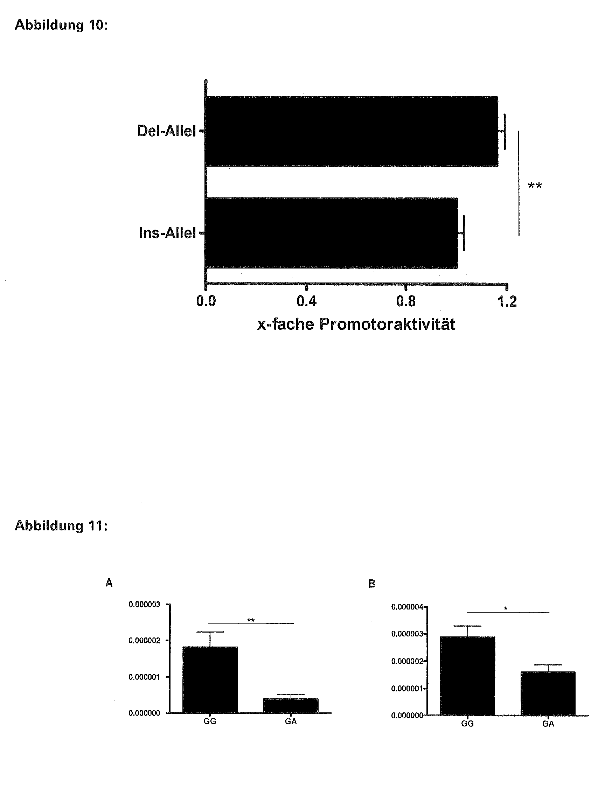

Es wurde untersucht, welche funktionellen Änderungen den Promotorpolymorphismen im Gen *CHK2* zuzuordnen sind. Denkbar sind hier beispielsweise eine Korrelation zu alternativem Spleißen, gewebespezifische Expression oder eine Überexpression des CHK2-Proteins in Abhängigkeit von Genotypen bzw. Haplotypen des Promotors von *CHK2.* Hierzu wurde zunächst mittels eines Computer-Programms untersucht, ob die gefundenen Nukleotidaustausche die Bindung von Transkriptionsfaktoren beeinflussen können. Transkriptionsfaktoren binden an spezifische Konsensus-Sequenzen und können die Promoteraktivität steigern oder vermindern, so dass eine verstärkte oder verminderte Transkription des Gens resultiert und somit die Expression des kodierten Proteins gesteigert oder vermindert wird. Wie in Abbildung 7 dargestellt, befinden sich alle oben erwähnten Promotor-SNPs in einer Konsensus-Sequenz für Bindungsstellen unterschiedlicher Transkriptionsfaktoren (z.B. p53, NFkB oder Mef2), deren Bindung durch die Polymorphismen beeinträchtigt werden kann. Das Auftreten bestimmter Genotypen führt zu einem Wegfall oder einer Neubildung dieser Bindungsstellen durch die Veränderung ihrer Konsensus-Sequenzen. Zur experimentellen Untersuchung dieses Effekts wird ein sog. EMSA (Electrophoretic mobility shift assay) durchgeführt. Bei diesem Versuch werden kurze Nukleinsäureabschnitte, die den jeweiligen Polymorphismus beinhalten, mit Zellkernextrakten inkubiert. Transkriptionsfaktor-Proteine, die sich in diesen Extrakten befinden, binden nun mit unterschiedlicher Intensität an die Nukleinsäureabschnitte. Die Bindung an die DNA wird schließlich im Röntgenfilm sichtbar gemacht. Dabei resultiert aus einer starken Bindung eine intensive Bande. Abbildung 8 zeigt das Resultat dieses Versuchs mit spezifischen Konstrukten, die entweder das C- oder das G-Allel des -7235C>G-Polymorphismus enthalten. Das Vorhandensein der C-Konstrukt-Bande beweist eine Bindung eines Transkriptionsfaktors an diese Region. Das G-Konstrukt weist diese Bande nicht auf, was zeigt, dass kein Transkriptionsfaktor an dieses Allel bindet. Die Abschwächung der Bandenintensität durch ein spezifisches Oligonukleotid zeigt, dass es sich bei dem bindenden Transkriptionsfaktor um eine spezifische Bindung handelt. Abbildung 9 zeigt das Resultat dieses Versuches mit spezifischen Konstrukten, die entweder das Insertions- oder das Deletionsallel des -10649-(-10621)del29-Polymorphismus enthalten. Beide Allele führen zu einer Bindung eines Transkriptionsfaktors, der jeweils durch das andere Allel kompetitiert werden kann. Hierbei handelt es sich aber um unterschiedliche Transkriptionsfaktoren. In Abbildung 10 wird zudem dargestellt, das die Bindung des Transkriptionsfaktors an das Deletionsallel zu einer stärkeren Promotoraktivierung führt als beim Insertionsallel.

Als nächstes wurde die Expression von *CHK2* auf mRNA-Ebene mittels Realtime-PCR in menschlichem Gewebe untersucht. Hierfür wurde mRNA aus menschlichem Operationsgewebe bei Mammakarzinom-Operationen gewonnen sowie aus Blutzellen von Leukämie-Patienten und mittels reverser Transkriptase in cDNA umgeschrieben. Das Verfahren ist dem Fachmann geläufig. Nachfolgend wurde das Expressionsniveau mittels Realtime-PCR (Taqman-Verfahren) bestimmt und mit dem Expressionsniveau des Housekeeping-Gens □-*Actin* abgeglichen.

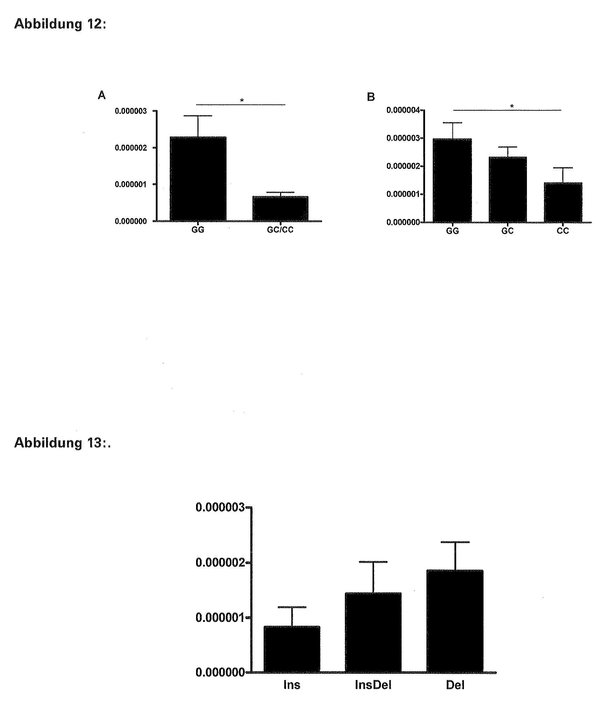

Die Ergebnisse sind in den Abbildungen 11 bis 13 dargestellt. Teilabbildung 11A zeigt, dass der GG-Genotyp des -7161G>A-SNPs eine signifikant höhere mRNA-Expression in Mammakarzinomen aufweist als der GA-Genotyp. Auch Abbildung 11B zeigt eine vermehrte mRNA-Expression für den GG-Genotyp in CLL-Patienten. Auch der Polymorphismus -7235C>G zeigt einen Allel-abhängigen Unterschied in der Genexpression. Wie Abbildungen 12A und 12B veranschaulichen, haben C-Allel-Träger deutlich weniger CHK2-mRNA als Träger des GG-Genotyps. Dies trifft für Mammakarzinom-Patientinnen und für CLL-Patienten zu. Abbildung 13 stellt eine vermehrte mRNA-Expression für die homozygote Deletion bei Mammakarzinom-Patientinnen dar. Die homozygote Insertion weist die niedrigste Expression auf. Der heterozygote Genotyp liegt dazwischen. Dabei ist ein Gen-Dosis-Effekt zu beobachten.

Damit wurde nachgewiesen, dass es im Gen *CHK2* Genveränderungen gibt, die eine Expressionsänderung von CHK2 im Karzinom-Gewebe bewirken. Dies können die oben beschriebenen Promotor-Polymorphismen sein oder Polymorphismen, die mit diesen SNPs im Kopplungsungleichgewicht stehen. Bestandteil der hier beschrieben Erfindung ist damit auch, die Expression von CHK2 zu quantifizieren, mit bekannten Polymorphismen des *CHK2* zu assoziieren und neue, noch besser geeignete Polymorphismen zu entdecken und zu validieren.

Die hier gezeigten Befunde einer Genotyp-abhängigen Expression von CHK2 in humanem Karzinom-gewebe ist überaus bedeutsam, da eine geringere Aktivität von CHK2 Mikrosatelliten- und chromosomale Instabilität hervorrufen kann, die beide mit zu den Kennzeichen genomischer Instabilität gehören und damit die Onkogenese begünstigen und sich negativ auf die Tumorprogression auswirken (Ahn et al. The CHK2 protein kinase. 2004 DNA Repair 3:1039-1047). Zudem kann sich diese Genotypabhängige Genexpression von *CHK2* auch auf das Ansprechen auf eine Therapie mit CHK2-Inhibitoren auswirken. Es ist zu erwarten, dass eine niedrige Genexpression prädisponiert durch einen bestimmten Genotyp, z.B. der CC-Genotyp des -7235C>G-Polymorphismus, stärker auf CHK2-Inhibitoren anspricht als andere Genotypen. Damit können Genveränderungen im Gen CHK2 dazu verwendet werden, das Ansprechen auf eine Krebstherapie vorherzusagen, um zum Beispiel Responder versus Non-Responder zu diskriminieren. Diese Genveränderungen können auch für eine Dosisfindung eingesetzt werden bzw. für die Vorhersage des Auftretens unerwünschter Arzneimittelwirkungen. Solche Krebstherapien können im weitesten Sinne medikamentös erfolgen, d.h. durch Zuführung von Substanzen in den Körper, oder diese Krebstherapeutika können physikalisch wirksam sein (wie Bestrahlung, Wärme, Kälte).

Wir erwarten somit die Beeinflussung von Krankheitsverläufen, insbesondere bei Tumorerkrankungen, sowie ein geändertes Ansprechen auf Substanzen, die die Regulationskaskade von CHK2 beeinflussen oder Substanzen, die direkt CHK2 inhibieren.

### Verwendung von Genveränderungen in CHK2 zur Vorhersage von Erkrankungsrisiken und Krankheitsverläufen

Aufgrund der Schlüsselfunktion der Checkpointkinase 2 für die Regulation des Zellzyklus ist es ein wesentlicher Bestandteil der Erfindung, dass unter Verwendung von Genveränderungen in *CHK2* generell Erkrankungsrisiken und Krankheitsverläufe vorhergesagt werden können.

Die Multistep-Entwicklung von Krebs spiegelt die Akkumulation genetischer Veränderungen wider, die zur Transformation von normalen Zellen zu Krebszellen und von normalem Gewebe zu benignen und evtl. malignen, invasiven Tumoren führen. Die Ansammlung von Alterationen in Tumorsuppressorgenen und Protoonkogenen beschleunigt die Tumorgenese und kann sowohl Radio- als auch Chemotherapie beeinflussen. Es wird allerdings immer deutlicher, dass gestörte DNA-Reparaturmechanismen sowie Checkpoints der Grund für die erhöhte genomische Instabilität von Tumoren sind. Da Checkpoints eine zentrale Rolle für die Erhaltung der genomischen Integrität besitzen, ist unmittelbar zu erwarten, dass der Verlauf vielfältiger und ganz unterschiedlicher Tumorerkrankungen bei einer genetisch determinierten, verminderten Aktivierbarkeit von Checkpoints beeinflusst wird. Dies bedeutet, dass durch Expressionsänderungen von Proteinen, die in allen menschlichen Körperzellen exprimiert werden und die Zelle vor DNA-Schäden bewahren, Zellfunktionen reguliert werden, die alle physiologischen und pathophysiologischen Vorgänge entscheidend beeinflussen oder zumindest modulieren. Daneben werden auch Antworten auf Pharmaka in besonderer Weise beeinflusst. Hiervon sind erwünschte, aber auch unerwünschte Arzneimittelwirkungen betroffen.

Es wurde in der wissenschaftlichen Literatur wiederholt postuliert, dass Funktionsveränderungen von Checkpoint-Proteinen einen nachhaltigen Einfluss auf vielfältige Erkrankungen bzw. auf den Verlauf vielfältiger Erkrankungen haben, da es sich um phylogenetisch hochkonservierte Pathways handelt. Solche Genveränderungen können strukturverändernde Mutationen in den Checkpointproteinen sein, die beispielsweise die Aktivierung der Proteine durch Phosphorylierung oder die Substratselektivität verringern. Ferner kann das Expressionsniveau verändert sein, wodurch die Initiation der nachfolgenden Reaktionskaskaden, die z.B. Apoptose induzieren, reduziert ist oder es können Spleißvarianten mit geänderter Funktion auftreten. All diese Veränderungen werden als genetische Prädisposition für Krebs angesehen.

Aus den genannten Beispielen geht hervor, dass
1. Genveränderungen in Genen, die für ubiquitär exprimierte Proteine kodieren, vielfältige Erkrankungen bzw. verursachen vielfältige Erkrankungsrisiken beeinflussen und
2. Checkpoint-Proteine Teil des komplexen Netzwerkes zur Aufrechterhaltung der genomischen Integrität im menschlichen Körper sind.

Erkrankungen, die mit einer Genveränderung in *CHK2* einhergehen, und beispielsweise durch ein geändertes Expressionsniveau des CHK2-Proteins bestimmt werden, sind beispielsweise benigne und maligne Neoplasien jedes Ursprungsgewebes

Ein wesentlicher Bestandteil der Erfindung ist folglich die Bereitstellung diagnostisch relevanter Genveränderungen im Gen *CHK2* als Prognosefaktor für Ile Krebserkrankungen des Menschen. Dies soll nachfolgend an ausgewählten beispielen verdeutlicht werden.

**Kolorektales Karzinom** - Das kolorektale Karzinom ist die häufigste Tumorart im Gastrointestinaltrakt und eine der Hauptursachen für einen tumorbedingten Tod weltweit (12-15% der gesamten Krebsmortalität). Die mittlere Fünfjahresüerlebens-ate nach der Tumorresektion beläuft sich auf nur 50%. Die Standardmethode zur Vorhersage des Krankheitsverlaufs ist das TNM- bzw. UICC-Stadiensystem. Patienten mit den UICC-Stadien III oder IV haben generell eine schlechtere Prognose als Patienten mit den UICC-Stadien I oder II. Eine adjuvante Chemotherapie wird bei metastasierten kolorektalen Karzinomen (UICC-Stadien III und IV) durchgeführt und kann den lokalen Effekt einer Strahlentherapie verstärken. Ein Großteil dieser Patienten entwickelt Rezidive oder Metastasen, was eine intensive Nachsorge erforderlich macht. Somit ist es wichtig, molekulare Marker zu identifizieren und zu etablieren, die den weiteren Verlauf der Erkrankung vorhersagen können. Ein weiterer Bestandteil der Erfindung besteht darin, Genveränderungen in *CHK2* zu nutzen, um den weiteren Verlauf des kolorektalen Karzinoms vorherzusagen.

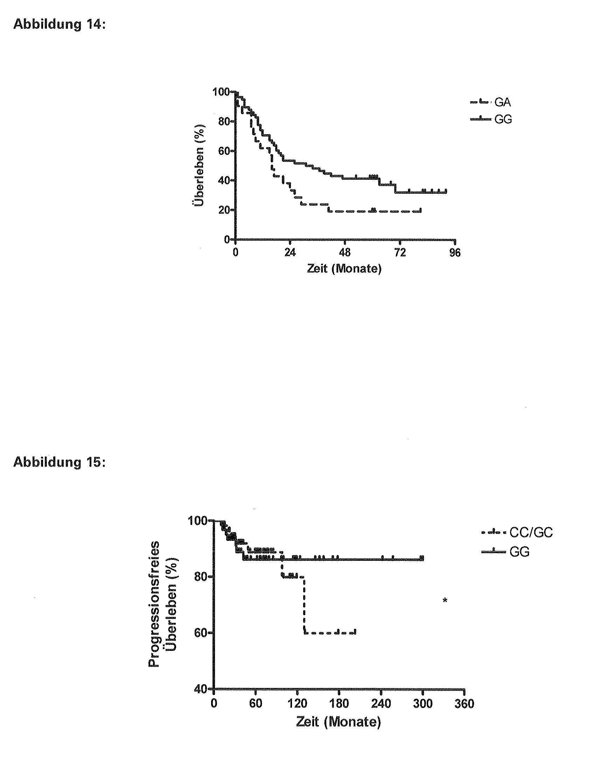

Abbildung 14 zeigt einen Unterschied bezüglich des Überlebens in Abhängigkeit vom -7161 G>A-Polymorphismus bei Patienten mit kolorektalen Tumoren der Stadien UICC III und IV. Patienten mit dem GG-Genotyp überleben hier länger als Patienten, die den heterozygoten GA Genotyp besitzen.

**Chronische lymphatische Leukämie -** Charakteristisch für die chronische lymphatische Leukämie (CLL) ist eine große Zahl von entarteten Lymphozyten. Insgesamt 30 % aller leukämischen Erkrankungen sind chronisch lymphatische Leukämien. Das mittlere Erkrankungsalter liegt bei 65 Jahren. Eine CLL kann bis zu 20 Jahre lang gutartig verlaufen, das heißt, die Patienten zeigen außer vergrößerten Lymphknoten, Müdigkeit und Appetitlosigkeit keine Symptome. Die Behandlung setzt erst dann ein, wenn die Zahl der Lymphozyten stark ansteigt, der Anteil der Erythrozyten und Thrombozyten absinkt oder andere Komplikationen auftreten. Eine frühzeitige Behandlung hat keinen Einfluss auf den Verlauf der Erkrankung. Die wichtigste therapeutische Maßnahme ist die Chemotherapie. Je weiter die Erkrankung fortgeschritten ist, desto größer sind die Gesundheitsstörungen durch die Veränderung des Organsystems. Je nach Binet-Stadium der Krankheit kann der Arzt die Prognose abschätzen. Das Stadium einer CLL ist unter anderem dadurch gekennzeichnet, wie viele Lymphozyten sich im Blut und Knochenmark befinden, wie groß Milz und Leber sind und ob eine Anämie vorliegt. Eine CLL führt zu Veränderungen im Immunsystem, so dass Menschen, die an dieser Krankheit leiden, stärker gefährdet sind, andere Arten von Krebs zu entwickeln. Bei gleichem Binet-Stadium zeigen Patienten jedoch ganz unterschiedliche Krankheitsverläufe. Aufgabe der Erfindung ist es zu zeigen, dass Genveränderungen im Gen *CHK2* geeignet sind, den Verlauf der CLL vorherzusagen.

Hierzu wurden Patienten mit CLL bezüglich der beschrieben Genveränderungen in *CHK2* genotypisiert und der Genstatus mit dem Progressionsfreien Überleben assoziiert. Abbildung 15 zeigt das Überleben abhängig vom -7235C>G-Polymorphismus. Patienten, die Träger des CC/GC-Genotyps sind, überleben länger als Patienten, die homozygot GG sind.

**Nierenzellkarzinom** - Bei Nierenkrebs sind die Heilungschancen abhängig von der Tumorgröße und von der Tumorausbreitung. Bei Patienten ohne Metastasen beträgt die 10-Jahres-Überlebensrate bis zu 80%, allerdings mit einer erheblichen interindividuellen Variabilität. Durch den heute weit verbreiteten Einsatz von Ultraschalltechnik werden viele der Tumoren bereits in einem frühen nicht metastasierten Stadium erkannt und können so rechtzeitig behandelt werden. Bei einem Vorliegen von Fernmetastasen ist es möglich, die operative Entfernung der Niere mit einer anschließenden Immuntherapie mit Interleukin-2 oder Interferon-Alpha zu kombinieren. Dadurch wird die körpereigene Krebszellabwehr gestärkt. Beim metastasierten Nierenzellkarzinom kann mit der Kombination von Interferon, Interleukin-2 und 5-Fluorouracil in Studien eine Ansprechrate von 36% erreicht werden. Derzeit gibt es keine prädiktiven Marker für das Überleben bei Patienten mit Nierenzellkarzinom.

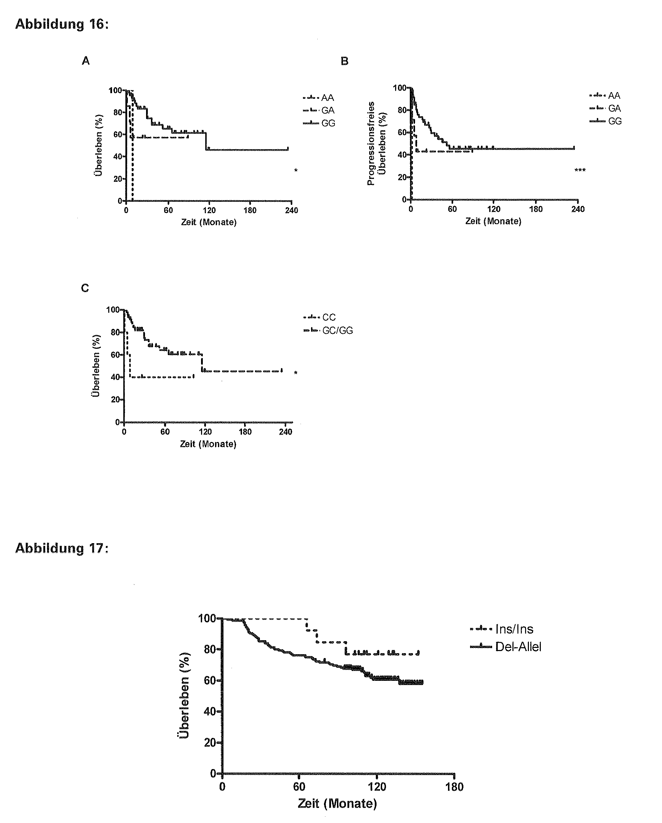

Abbildung 16A zeigt das Überleben in Abhängigkeit vom -7161G>A-Polymorphismus. Patienten mit dem GG-Genotyp überleben deutlich länger als Patienten mit den GA- und AA-Genotypen (p < 0,05). Die mediane Zeit bis zum Tod beträgt bei GG-Trägern 115 Monate, für Patienten mit dem AA-Genotyp dagegen nur 9 Monate. Ähnliches gilt auch für das Progressionsfreie Überleben abhängig vom - 7161 G>A-Polymorphismus (Abbildung 16B). Auch hier zeigen die Patienten mit dem GG-Genotyp die geringste Progression über den Beobachtungszeitraum. Das mediane Progressionsfreie Überleben beträgt für GG 52 Monate, für AA dagegen nur 2 Monate (p < 0,001). Der -7235C>G-Polymorphismus war ebenfalls mit dem Überleben assoziiert (Abbildung 16C). Die G-Allelträger wiesen ein längeres Überleben auf als Patienten mit dem CC-Genotyp (p < 0,05).

**Mammakarzinom** - Das Mammakarzinom ist der häufigste Tumor der weiblichen Bevölkerung in Europa und den USA. Es betrifft 7 bis 10% aller Frauen und macht 25% der gesamten weiblichen Krebsmortalität aus. Die Ätiologie des Mammakarzinoms ist noch nicht bekannt, jedoch sind Risikofaktoren beschrieben, wie eine familiäre Disposition, Strahlenexposition oder Östrogeneinfluss. Die meisten Patientinnen haben ein invasives Karzinom. Mit wenigen Ausnahmen wird jedes operable Mammakarzinom selbst bei nachgewiesener Fernmetastasierung chirurgisch behandelt. Die unterschiedlich radikale operative Erstversorgung hat Variationen der lokoregionären Rezidivrate, nicht aber der langfristigen Überlebenschance zur Folge. Zudem können Rezidive oder Fernmetastasen nicht selten erst nach 5 oder sogar 10 Jahren manifest werden. Es ist deshalb wichtig, diese Läsionen frühzeitig zu erkennen und die Patientinnen engmaschig nachzusorgen.

Nachsorgeuntersuchungen werden in regelmäßigen Intervallen durchgeführt, bei zwischenzeitlichem Verdacht auch bis zu 10 Jahre postoperativ. Bislang gibt es kaum valide Marker, die für den weiteren Verlauf der Erkrankung prädiktiv sind. Derzeit werden daher die klassischen Faktoren wie Tumorgröße, Metastasierung, Lymphknotenbefall, Hormonrezeptorstatus etc. für die Prognose herangezogen. Genetische Marker für Überlebenswahrscheinlichkeit und Therapieansprechen würden die Betreuung von Patientinnen mit Mammakarzinom wesentlich verbessern. Aufgabe der Erfindung ist es zu zeigen, dass die Verwendung von Genveränderungen in *CHK2* dazu geeignet ist, den weiteren Verlauf der Erkrankung vorherzusagen.

Abbildung 17 zeigt das Überleben von Mammakarzinom-Patientinnen in Abhängigkeit vom -10649-(-10621)del29-Polymorphismus. Patientinnen mit der homozygoten Insertion zeigen das beste Überleben verglichen mit Patientinnen, die mindestens ein Deletions-Allel tragen.

**Glioblastom -** Gliome kommen hauptsächlich im Erwachsenenalter vor. Die Ätiologie ist unbekannt. Das histologische Bild der Gliome ist geprägt durch maligne Progression, diffuse Invasion und starke Heterogenität. Das häufigste maligne Gliom ist das Glioblastom (WHO Grad IV). Es breitet sich häufig über den Balken in die andere Hirnhälfte aus. Nur bei Grad I-Gliomen (pilozytischen Astrozytomen) ist eine kurative operative Therapie möglich. Alle anderen Gliome rezidivieren. Sie sind bislang nicht heilbar. Die mediane Überlebenszeit beim Glioblastom beträgt 6 bis 8 Monate. Ohne postoperative Bestrahlung beträgt die mediane Überlebenszeit nur 2 bis 3 Monate. Bisher gibt es keine Marker, die für den Verlauf dieser schweren Erkrankung prädiktiv sind.

Genetische Marker für Überlebenswahrscheinlichkeit und Therapieansprechen würden die Betreuung von Patienten mit Glioblastom wesentlich verbessern. Aufgabe der Erfindung ist es zu zeigen, dass die Verwendung von Genveränderungen in *CHK2* dazu geeignet ist, den weiteren Verlauf der Erkrankung vorherzusagen.

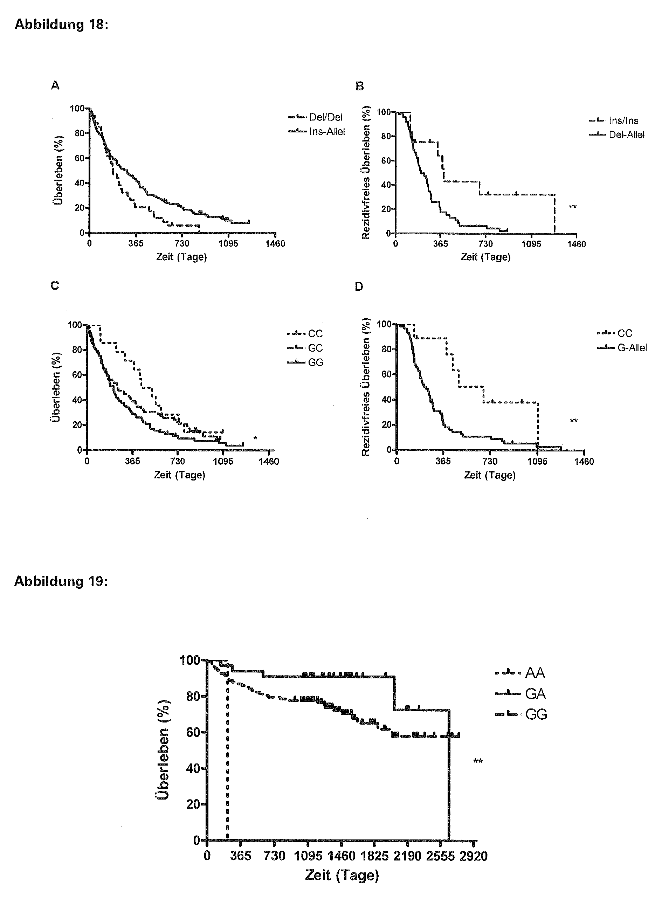

Die Abbildungen 18A und B zeigen, dass der -10649-(-10621)del29-Polymorphismus einen Einfluss auf den Krankheitsverlauf beim Glioblastom hat.

Patienten mit dem Insertionsallel zeigen ein längeres Überleben und ein ebenfalls längeres Rezidivfreies Überleben. Im Median bleiben Patienten, die homozygot für die Insertion sind, 390 Tage rezidivfrei, aber Patienten mit dem Deletionsallel nur 206 Tage. Der -7235C>G-Polymorphismus korreliert ebenfalls mit dem Krankheitsverlauf (Abbildungen 18C und D). Patienten mit dem CC-Genotyp überleben länger als Patienten mit dem GG-Genotyp und heterozygote Individuen liegen dazwischen, was für einen Gen-Dosis-Effekt spricht. Patienten mit dem CC-Genotyp bleiben ebenfalls am längsten rezidivfrei verglichen mit Patienten, die das G-Allel tragen.

**Prostatakarzinom** - Das Prostatakarzinom ist das zweithäufigste männliche Malignom. Es macht 9-11 % aller Tumorerkrankungen aus, wobei die Inzidenz steigt. Mehr als 50% der Fälle betreffen Patienten älter als 70 Jahre. Zur Früherkennung ist eine Bestimmung des Prostataspezifischen Antigens (PSA) möglich. Solche Untersuchungen zur Früherkennung sind sinnvoll bei Männern älter als 50 Jahre mit einer Lebenserwartung von mehr als 10 Jahren. Die Aussagekraft des PSA-Wertes ist allerdings umstritten. Das PSA muss vor der rektalen Untersuchung abgenommen werden, da sonst die PSA-Werte falsch-hoch sind. Zudem können erhöhte PSA-Werte auch bei einer benignen Prostatitis vorkommen. In den Frühstadien ist das Prostatakarzinom meist asymptomatisch, da es sich harnröhrenfern entwickelt. Daher gibt es keine Möglichkeit der Selbstkontrolle zur Früherkennung. Die Fünfjahresüberlebensrate beträgt bei einem Prostatakarzinom mit Flocks-Stadium C 60%, nach 10 Jahren ist die Überlebenswahrscheinlichkeit jedoch nur noch 30% und nach 15 Jahren nur noch 20%. Bislang gibt es kaum valide Marker, die für den weiteren Verlauf der Erkrankung prädiktiv sind.

Genetische Marker für Überlebenswahrscheinlichkeit und Therapieansprechen würden die Betreuung der Patienten wesentlich verbessern. In Abbildung 19 ist das Überleben abhängig vom -7161G>A-Polymorphismus dargestellt. Patienten mit mindestens einem G-Allel zeigen ein besseres Überleben als Patienten mit dem AA-Genotyp. Während das mediane Überleben bei G-Allelträgern 2650 Tage beträgt, sind es bei Trägern des AA-Genotyps nur 220 Tage.
Diese Ergebnisse belegen eindeutig die Verwendbarkeit der Genveränderungen im Gen *CHK2* für den hier beschriebenen Zweck. Diese Erkrankungen stehen *a priori* in keinerlei Zusammenhang.

### Verwendung von Genveränderungen im Gen CHK2 zur Vorhersage von Krankheitsverläufen und Therapieansprechen

Pharmakogenetik im Sinne der Erfindung beschäftigt sich mit der Diagnostik der Wirksamkeit von Pharmaka, der Potenz und Effizienz von Pharmaka und dem Auftreten unerwünschter Wirkungen Die Wirksamkeit von Arzneimitteln und/oder das Auftreten unerwünschter Nebenwirkungen werden neben den spezifischen Stoffeigenschaften der chemisch definierten Produkte durch eine Reihe von Parametern definiert. Zwei wichtige Parameter, die erzielbare Plasmakonzentration und die Plasmahalbwertszeit, bestimmen in hohem Maß die Wirksamkeit oder Unwirksamkeit von Pharmaka oder das Auftreten unerwünschter Wirkungen. Die Plasmahalbwertszeit wird unter anderem dadurch bestimmt, mit welcher Geschwindigkeit bestimmte Pharmaka in der Leber oder anderen Körperorganen zu wirksamen oder unwirksamen Metaboliten verstoffwechselt und mit welcher Geschwindigkeit sie aus dem Körper ausgeschieden werden, wobei die Ausscheidung über die Nieren, über die Atemluft, über den Schweiß, über die Spermaflüssigkeit, über den Stuhl oder über andere Körpersekrete erfolgen kann. Daneben wird die Wirksamkeit bei oraler Gabe durch den sog. "first-pass-Effekt" limitiert, da nach Resorption von Pharmaka über den Darm ein bestimmter Anteil in der Leber zu unwirksamen Metaboliten verstoffwechselt wird.

Mutationen oder Polymorphismen in Genen metabolisierender Enzyme können die Aktivität derselben in der Weise verändern, dass deren Aminosäurezusammensetzung verändert wird, wodurch die Affinität zum zu metabolisierenden Substrat erhöht oder erniedrigt wird und damit der Metabolismus beschleunigt oder verlangsamt sein kann. In ähnlicher Weise können Mutationen oder Polymorphismen in Transportproteinen die Aminosäurezusammensetzung in der Weise verändern, dass der Transport und damit die Ausscheidung aus dem Körper beschleunigt oder verlangsamt werden.

Zur Auswahl der für einen Patienten optimal geeigneten Substanz, der optimalen Dosierung, der optimalen Darreichungsform und zur Vermeidung unerwünschter, z. T. gesundheitsschädlicher oder tödlicher Nebenwirkungen ist die Kenntnis von genetischen Polymorphismen oder von Mutation, die zur Änderung der Genprodukte führen, von herausragender Bedeutung.

### Die Bedeutung der Checkpointkinse 2 für Chemotherapeutika und Bestrahlung

Genetische Instabilität ist ein Kennzeichen von allen Tumoren und spielt auch bei der Onkogenese, Progression und der Entwicklung von Resistenzen gegenüber Pharmaka eine Rolle. Die meisten Tumorzellen besitzen einen defekten G1-S-Checkpoint, was ihnen einen Überlebensvorteil bringt. Dieser Defekt allerdings bedingt, dass Tumorzellen sehr abhängig vom G2-Checkpoint sind, wenn Stimuli vorhanden sind, die die genomische Integrität bedrohen. CHK2 ist verantwortlich für die Aufrechterhaltung des G1-Checkpoints, wenn DNA-Schäden auftreten. Somit bieten die Aktivierung von CHK2, und damit das Wiedereinsetzen des G1-Checkpoints, die Möglichkeit, Therapieresistenzen zu umgehen. Es wurde bereits gezeigt, dass der Wegfall von CHK2 eine Resistenz gegenüber Bestrahlung bedingt. Diese könnte über CHK2-Aktivatoren aufgehoben werden. Da CHK2 auch den G2-Checkpoint beeinflusst, kann somit ihre Hemmung und damit der Wegfall des G2-Checkpoints die Möglichkeit bieten, dass sich DNA-Schäden und -veränderungen ansammeln können, die durch genotoxische Substanzen und Bestrahlung bedingt werden, und die Tumorzelle daran stirbt.

Treten Genveränderungen in *CHK2* auf, die die Genexpression beeinflussen, so hat dies Auswirkungen auf die Wirksamkeit dieser CHK2-Inhibitoren. Es ist zu erwarten, dass Patienten mit einer genotypabhängigen niedrigeren *CHK2*-Expression besser auf die Inhibitoren ansprechen als Patienten mit einer höheren *CHK2*-Expression. Zudem bedeutet es, dass die Kombinationstherapie von CHK2-Inhibitoren mit Chemo- und Immuntherapeutika und/oder Bestrahlung beeinflusst werden kann. Dasselbe gilt für CHK2-Aktivatoren. Es ist zu erwarten, dass Patienten mit einer Genotyp-abhängigen höheren *CHK2*-Expression besser auf Aktivatoren ansprechen als Patienten mit einer niedrigeren *CHK2-*Expression. Daraus ergibt sich die Möglichkeit einer individuellen Diagnostik der generellen Ansprechbarkeit auf diese Krebstherapeutika und Therapiemaßnahmen sowie eine individuelle Vorhersage des Risikos unerwünschter Wirkungen durch diese Therapien.

### Die genotypabhängige Diagnostik der Expression von CHK2 erlaubt eine generelle Diagnostik der Wirksamkeit von Chemotherapeutika und Bestrahlung, deren optimale Dosierung und das Auftreten von Nebenwirkungen.

Die Chemotherapie verwendet solche Stoffe, die ihre schädigende Wirkung möglichst gezielt auf bestimmte Krebszellen ausüben und diese abtöten oder in ihrem Wachstum hemmen. Eine bestimmte Zytostatikadosis kann immer nur einen bestimmten Anteil der Zielzellen töten, der mit fortschreitender Behandlung gleich bleibt. Deshalb darf eine Chemotherapie im Laufe der Behandlung nicht vermindert werden, auch wenn der Tumor bereits nicht mehr nachweisbar ist. Es muss vielmehr damit gerechnet werden, dass durch eine schwache Behandlung gerade die widerstandsfähigen Tumorzellklone selektiert werden. Die Chemotherapie wird in schneller Abfolge appliziert, und fast immer werden zwei oder mehr Zytostatika kombiniert, um die Wirksamkeit zu erhöhen. Die Therapie ruft dadurch auch Nebenwirkungen hervor, die nach den Common Toxicity Criteria klassifiziert werden. Diese Kriterien beinhalten: Anzahl der Leukozyten und Thrombozyten, Übelkeit, Erbrechen, Durchfall und Stomatitis.

Unter Radiotherapie versteht man die Anwendung ionisierender hochenergetischer Strahlen, um bösartige Tumorerkrankungen zu heilen. Solche maligne Tumoren werden oft auch kombiniert chemo- und radiotherapeutisch behandelt. Eine Vielzahl an Tumorerkrankungen kann so auch in fortgeschrittenen Stadien geheilt werden. Um die Nebenwirkungen gering zu halten, wird die Bestrahlung auf viele tägliche Einzeldosen aufgeteilt und über mehrere Wochen verabreicht. Trotzdem treten Nebenwirkungen, wie Rötungen Übelkeit, Durchfälle oder Haarausfall, in Abhängigkeit von Dosis, Eindringtiefe und Anzahl der Einzeldosen auf.

Der Erfindung liegt nun zugrunde, dass ein Verfahren entwickelt wurde, das generell zur Diagnostik der Aktivierbarkeit der Checkpointkinase 2 und damit verbunden dem G1- und dem G2-Checkpoint geeignet ist. Hierzu werden eine oder mehrere Polymorphismen im Gen *CHK2* untersucht. Bei einer hohen Expression kommt es vorhersagbar zu einer gesteigerten Aktivierbarkeit der Checkpoints und damit zu ausreichend Zeit, um Reparaturmechanismen an der DNA nach Schädigung durchzuführen. Bei einer niedrigen *CHK2*-Expression sind die Checkpoints weniger aktivierbar und DNA-Schäden werden nicht oder nicht ausreichend repariert. Damit erlaubt eine Bestimmung des Vorliegens von Polymorphismen in *CHK2* die Diagnostik der Wirksamkeit und unerwünschten Wirkungen von Arzneimitteln, insbesondere Zytostatika, sowie anderer Therapieformen, die das Erbgut der Tumorzellen schädigen, z. B. Bestrahlung. Daneben können solche Polymorphismen in *CHK2* dazu verwendet werden, die Wirkungen von Pharmaka zu diagnostizieren, die mit einem CHK2-Inhibitor kombiniert werden. Zusätzlich kann die Diagnostik des Allel- oder Haplotypstatus in *CHK2* dazu eingesetzt werden, die individuell optimale und verträgliche Dosierung von Arzneimitteln zu ermitteln.

Zur Diagnostik einer gesteigerten oder reduzierten Aktivierbarkeit der Checkpointkinase 2 und der Checkpoints dient insbesondere der Nachweis der hier beschriebenen CHK2-Promotorpolymorphismen, entweder alleine oder in allen denkbaren Kombination.

Daneben können zur Diagnostik alle weiteren Genveränderungen in *CHK2* verwendet werden, die in einem Kopplungsungleichgewicht zu diesen Polymorphismen stehen und/oder den alternativen Spleißvorgang oder die Expression zusätzlich fördern oder hemmen.

Diese Genveränderungen können mit beliebigen, dem Fachmann geläufigen Verfahren nachgewiesen werden, z.B. direkte Sequenzierung, Restriktionsanalyse, reverse Hybridisierung, Dot-blot- oder Slotblot-Verfahren, Massenspektrometrie, Taqman®- oder Light-Cycler®-Technologie, Pyrosequencing etc. Ferner können diese Genpolymorphismen gleichzeitig nach Mulitplex-PCR und Hybridisierung an einen DNA-Chip detektiert werden. Daneben können zur Diagnostik einer gesteigerten Aktivierbarkeit von G-Proteinen auch andere Verfahren eingesetzt werden, die den direkten Nachweis des Expressionsniveaus von CHK2 oder Spleißvarianten von CHK2 ermöglichen.

Das genannte Verfahren eignet sich insbesondere zur Diagnostik der Wirkung von Substanzen, die die DNA der Tumorzellen schädigen. Zu diesen Substanzen gehören Oxaliplatin, 5-Fluorouracil, Folinsäure, Irinotecan, Capecitabin und Cisplatin, wobei die Liste beliebig verlängert werden kann. Vorhergesagt werden können daneben die Wirkungen von Immuntherapeutika (z.B. Interferone oder Interleukine) oder Inhibitoren der Signaltransduktion in Tumorzellen.

Vorhergesagt werden können ferner die Wirkungen von radiotherapeutischen Maßnahmen, wie Gamma-, Röntgenstrahlung, Elektronen, Neutronen, Protonen und Kohlenstoffionen, wobei die Liste beliebig fortgeführt werden kann. Im weiteren Sinne wird unter Strahlentherapie auch die medizinische Anwendung von Mikrowellen- und Wärmestrahlen, die Licht- und UV-Therapie sowie die Behandlung mit Ultraschallstrahlung verstanden.

Die folgenden Beispiele dienen der weiteren Erläuterung der Verwendung von Genveränderungen in *CHK2* zur Vorhersage von Erkrankungsrisiken und Krankheitsverläufen.

### Beispiele

Dazu wurden verschiedene Tumorkollektive und gesunde Kontrollen für die *CHK2*-Polymorphismen genotypisiert und die Genotyp- und/oder Allelverteilungen verglichen. Hierbei stellten sich signifikante Unterschiede bei der Verteilung der Genotypen und Allele heraus. Es kann nicht generell vorhergesagt werden, ob eine erhöhte Expression von CHK2 günstig oder ungünstig ist. Damit eignen sich die Polymorphismen in *CHK2,* ein Erkrankungsrisiko vorherzusagen. Das Erkrankungsrisiko für das Risikoallel oder für Risikogenotypen (alleine oder kombiniert) wird als "odds ratio" (OR) zusammen mit dem 95 % Konfidenzintervall (95 % CI) und dem p-Wert angegeben.

### Beispiel 1

### Patienten mit kolorektalem Karzinom (n=143) vs. Kontrollen (n=235)

**-7161G>A -7235C>G**

| **Allel** | **Kolonkarzinom** | **Kontrollen** | | **Geno-typ** | **Kolonkarzinom** | **Kontrollen** |
|---|---|---|---|---|---|---|
| G | 241 (84.3%) | 419 (89.2%) | | CC | 8 (5.6%) | 25 (10.7%) |
| A | 45 (15.7%) | 51 (10.8%) | | CG | 70 (49.0%) | 88 (37.6%) |
| | | | | GG | 65 (45.5%) | 121 (51.7%) |
| P=0,050 | | | P=0,048 | | | |

Die Allel- oder Genotypverteilungen sind signifikant unterschiedlich (p = 0,05 bzw. 0,048);
OR A versus G = 1,534 (95 %CI = 0,997-2,361) p= 0.05;OR A versus G = 1,534 (95 %CI = 0,997-2,361) p= 0.05; OR CG versus CC = 2.486 (95%CI = 1,056-5,851) p = 0.034; OR CG versus CC = 2.486 (95%CI = 1,056-5,851) p = 0.034;

Damit kann -7161A-Allelträgern bzw. -7235 CG Genotypen ein erhöhtes Risiko zugeordnet werden, an einem Kolonkarzinom zu erkranken. Eine verminderte Expression von CHK2-mRNA ist also mit einem erhöhten Erkrankungsrisiko assoziiert.

### Beispiel 2

### Patienten mit Chronischer lymphatischer Leukämie (CLL, n=166) vs. Kontrollen (n=234)

**-7235C > G**

| **Genotyp** | **CLL** | **Kontrollen** |
|---|---|---|
| CC | 7 (4.2%) | 25 (10.7%) |
| CG | 75 (54.2%) | 88 (37.6%) |
| GG | 84 (50.6%) | 121 (51.7%) |

Die Genotypverteilungen sind signifikant unterschiedlich (p = 0,039). Es lassen sich die folgenden Risiken (OR) für eine CLL errechnen:
OR CG versus CC = 3,044 (95%CI = 1,246 - 7,435), P=0.012;
OR GG plus CG versus CC = 2,7 (95 % CI = 1,2 - 6,4) P = 0.024;

### Beispiel 3

### Patienten mit Glioblastom (n=198) vs. Kontrollen (n=235)

**-7161G>A**

| **Allel** | **Glioblastom** | **Kontrollen** | | **Geno-typ** | **Glioblastom** | **Kontrollen** |
|---|---|---|---|---|---|---|
| G | 331 (83.6%) | 419 (89.2%) | | GG | 145 (73.2%) | 188 (80.0%) |
| A | 65 (16.4%) | 51 (10.8%) | | GA | 41 (20.7%) | 43 (18.3%) |
| | | | | AA | 12 (6.1%) | 4 (1.7%) |

| | | | | | | |
|---|---|---|---|---|---|---|
| | | | | | | |

Die Allel- und Genotypverteilungen sind signifikant unterschiedlich (P=0,017 bzw. p=0,039) Die folgenden Risiken lassen sich errechnen:
OR A versus G: 1.613 (95% Cl = 1,088-2,393); p = 0,021;
OR AA versus GG: 3.890 (95% CI = 1.229-12.31); P=0,019;
OR AA plus AG versus GG: 3,73 (95% CI = 1,2 -11,8); P=0,021;

Damit kann -7161A-Allelträgern bzw. -7161AA/AG Genotypen ein erhöhtes Risiko zugeordnet werden, an einem Glioblastom zu erkranken. Eine verminderte Expression von CHK2-mRNA ist also mit einem erhöhten Erkrankungsrisiko assoziiert.

### Beispiel 4

### Patientinnen mit Mammakarzinom (n=240) vs. weibliche Kontrollen (n=78)

**-10649-(-10621)del29**

| **Genotyp** | **Mammakarzinom** | **Kontrollen** |
|---|---|---|
| II | 29 (12.1%) | 21 (26.9%) |
| ID | 162 (67.5%) | 38 (48.7%) |
| DD | 49 (20.4%) | 19 (24.4%) |

Die Genotypverteilung ist signifikant unterschiedlich /p =0,003). Die folgenden Risiken lassen sich errechnen: OR ID versus II: 3,087 (95% CI=1,590 - 5,995), p=0.001

## Patentansprüche

1. In vitro-Verfahren zur Vorhersage des Krankheitsrisikos für eine Krebserkrankung, des Krankheitsverlaufs, des Arzneimitteinutzen und Arzneimittelrisikos bei der Behandlung einer Krebserkrankung, **dadurch gekennzeichnet, dass** man in einer Patientenprobe nach einer oder mehreren Genveränderung/en in der Promotorregion des Gens CHK2 auf dem Humanchromosom 22q12.1 sucht und die Genveränderung ausgewählt ist aus dem Polymorphismus -7161G>A, dem Polymorphismus -7235C>G, dem Polymorphismus -10532G>A und dem Polymorphismus -10649-(-10621)del29.

2. Verfahren nach Anspruch 1, **dadurch gekennzeichnet, dass** man in einer Patientenprobe nach einem, zwei, drei oder vier der Polymorphismen -7161G>A, -7235C>G, -10532G>A und - 10649-(-10621)del29 sucht.
